# EUROPEAN PATENT APPLICATION

(11) **EP 4 249 505 A1**
(43) Date of publication of application: **27.09.2023**
(21) Application number: 20966375.6
(22) Date of filing: 23.12.2020
(51) Int. Cl.: C07K 14/605, A61K 38/26, C07K 1/04, A61P 1/16, A61P 3/04, A61P 3/06, A61P 3/10

(54) **LONG-ACTING GLUCAGON DERIVATIVE**

(71) Applicant: Zhejiang Doer Biologics Co., Ltd., Hangzhou, Zhejiang 310018 (CN); Zhejiang Heze Pharmaceutical Technology Co., Ltd., Hangzhou, Zhejiang 310018 (CN)
(72) Inventor: HUANG, Yanshan, Hangzhou, Zhejiang 310018 (CN); NI, Sheng, Hangzhou, Zhejiang 310018 (CN); XIA, Jinqiang, Hangzhou, Zhejiang 310018 (CN); ZHU, Mingyue, Hangzhou, Zhejiang 310018 (CN); FANG, Chen, Hangzhou, Zhejiang 310018 (CN); SUN, Peng, Hangzhou, Zhejiang 310018 (CN); ZHAO, Hang, Hangzhou, Zhejiang 310018 (CN); XU, Bingyong, Hangzhou, Zhejiang 310018 (CN)
(74) Representative: Canzler & Bergmeier Patentanwälte Partnerschaft mbB
(86) International application number: PCT/CN2020/138597
(87) International publication number: WO 2022/133797

(57) **Abstract**

Provided is a long-acting glucagon derivative, the amino acid sequence thereof comprising the sequence shown in SEQ ID NO: 9. Animal experiments show that, when compared to the similar fatty acid-modified GLP-1R single agonist Semaglutide (J Med. Chem. 2015, 58(18):7370-80), the long-acting glucagon derivative has stronger glucose tolerance and weight reduction activity in mice, and can last for a longer period of time.

## Description

### FIELD OF DISCLOSURE

The present invention relates to the field of biotechnology, and specifically to a long-acting glucagon derivative.

### DESCRIPTION OF RELATED ARTS

Glucagon-like peptide-1 (GLP-1) is a small peptide processed in specific tissues from proglucagon expressed *in vivo,* which has a molecular weight of about 3 kDa. In human body, selective cleavage of proglucagon molecules produces two biologically active forms of GLP-1, which are GLP-1(7-37)-OH and GLP-1(7-36)-NH2, respectively. The latter form of 30 amino acids accounts for the major part of the body at a proportion of about 80%. The physiological effects of GLP-1 are as follows: stimulating the proliferation and regeneration of pancreatic β-cells, preventing the apoptosis of pancreatic β-cells; promoting the secretion of insulin, inhibiting the secretion of pancreatic trypsin and esterase, inhibiting the secretion of glucagon, weakening the gastrointestinal motility, inhibiting gastric emptying, reducing appetite, reducing food absorption, and protecting the cardiovascular system, etc. It can be used for the treatment of type 2 diabetes and obesity in clinic. Compared with insulin, GLP-1 has a lower risk of causing hypoglycemia.

Glucagon (GCG for short), as a hormone that regulates blood glucose in the body, is also processed in tissues from proglucagon. Glucagon has a molecular weight of about 3 kDa and a biologically active form of GCG-(1-29)OH. As the only hormone in the body that raises blood glucose, glucagon can be used to raise blood glucose levels in hypoglycemic conditions, promote lipolysis, and increase satiety. Combined use of GCG and hypoglycemic drugs can increase energy consumption and reduce body weight (Physiol Rev 97:721-766, 2017).

Glucose-dependent insulinotropic peptide, also recognized as gastric inhibitory peptide (GIP), is a physiological incretin hormone, which can lower the blood glucose by stimulating the secretion of insulin and inhibiting the secretion of gastric acid.

GLP-1, GCG and GIP are all processed from proglucagon, so there is a high homology in sequence, collectively referred to as incretin hormones. It has been reported that the activity of GLP-1 or GIP can be enhanced by modifying the GCG sequence. For example, the modification of GCG in patents WO2011075393 and WO2012177444 produces GCG/GLP-1 dual-active hybrid peptide, the modification of GCG in patent WO2013192130 produces GCG/GIP dual-active hybrid peptide, and WO2015067716 mentioned that GCG/GLP-1/GIP triple active hybrid peptide was obtained based on the modification of GCG. However, such dual-active or even multi-active hybrid peptide drugs have not been approved for marketing so far. In addition, dual-active and multi-active hybrid peptide drugs currently under development generally have an extremely high biologically activity. However, it is well known that the GLP-1R agonist has side effects such as nausea, vomiting, diarrhea, nervousness, abdominal pain, etc. Clinical patients may even withdraw from clinical trials due to severe side effects. Therefore, it is still of profound significance to develop a product with fewer side effects and equivalent or even more superior efficacy.

### SUMMARY OF THE PRESENT INVENTION

In view of the disadvantages of the prior art above, the present invention is intended to provide a long-acting glucagon derivative to solve the problems in the prior art.

In order to achieve the above and other related objects, the first aspect of the present invention provides a long-acting glucagon derivative whose amino acid sequence includes a sequence of SEQ ID NO. 9 shown as below:
HX₂QGTFTSDX₁₀SKX₁₃X₁₄DSQAAQDFVQWLLAGGPSSGAPPPS-NH₂ (SEQ ID No. 9);
wherein, X₂ represents Aib;
X₁₀ represents Y, or K conjugated to a first long-acting carrier;
X₁₃ represents Y, or Aib;
X₁₄ represents L, or K conjugated to a second long-acting carrier.

In some embodiments of the present invention, the first long-acting carrier and/or the second long-acting carrier is a fatty acid chain.

In some embodiments of the present invention, the carbon-chain length of the fatty acid chain is 5-30, preferably 8-30;
and/or, the chemical structural formula of the fatty acid chain is shown as one of: 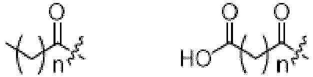 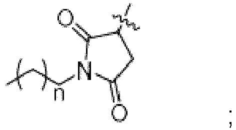
wherein, each n is independently 3 to 28, preferably 6 to 28, more preferably 17, 18 or 19.

In some embodiments of the present invention, the fatty acid chain is conjugated to K via a linker, the linker is one or more selected from the group consisting of -Abu-, -GABA-, -EACA-, -β-Ala-, -γGlu-, -D-γGlu- or dipeptide thereof, -β-Ala-β-Ala-, -γGlu-γGlu- and stereoisomeric forms thereof, -5-Aminopentanoyl-, -8-Aminooctanoyl-, -9-Aminononanoyl-, -10-Aminodecanoyl-, -OEG-, -2xOEG-, -γGlu-OEG-, -γGlu-2xOEG-, -D-γGlu-2xOEG-, -2xOEG-γGlu-, -γGlu-3xOEG-, -γGlu-8xPEG-, -γGlu-3xOEG-γ-Glu-8xPEG-.

In some embodiments of the present invention, X₁₃ represents Y, X₁₄ represents L, X₁₀ represents K conjugated to a fatty acid chain, and the linker is -2xOEG-γGlu-;
and/or, X₁₃ represents Aib, X₁₄ represents L, X₁₀ represents K conjugated to a fatty acid chain, and the linker is -2xOEG-γGlu-;
and/or, X₁₃ represents Aib, X₁₀ represents Y, X₁₄ represents K conjugated to a fatty acid chain, and the linker is -2xOEG-γGlu-.

In some embodiments of the present invention, the amino acid sequence of the long-acting glucagon derivative includes a sequence as shown in one of SEQ ID Nos. 6 to 8.

The second aspect of the present invention provides a method for preparing the long-acting glucagon derivative above, including: preparing the long-acting glucagon derivative through solid-phase synthesis.

The third aspect of the present invention provides a use of the above long-acting glucagon derivative in preparing a medicament, wherein the medicament is preferably for treatment of metabolic diseases.

In some embodiments of the present invention, the metabolic disease is selected from diabetes mellitus, obesity, dyslipidemia, nonalcoholic fatty liver disease, nonalcoholic steatohepatitis, other diabetes mellitus-related metabolic syndromes, high triglycerides, low HDL cholesterol and high LDL cholesterol, insulin resistance, obesity or glucose intolerance.

The fourth aspect of the present invention provides a pharmaceutical composition, including a therapeutically effective amount of the above long-acting glucagon derivative.

### BRIEF DESCRIPTION OF THE DRAWINGS

Fig. 1 shows a schematic representation of the mass spectrometry results of P13F in Embodiment 2 of the present invention.
Fig. 2 shows a schematic representation of the mass spectrometry results of P16F in Embodiment 3 of the present invention.
Fig. 3 shows a schematic representation of the mass spectrometry results of P29F in Embodiment 4 of the present invention.
Fig. 4 shows a schematic representation of the *in vitro* hGLP-1R agonist activity of the glucagon derivative in Embodiment 5 of the present invention.
Fig. 5 shows a schematic representation of the *in vitro* hGIPR agonist activity of the glucagon derivative in Embodiment 5 of the present invention.
Fig. 6 shows a schematic representation of the *in vitro* hGCGR agonist activity of the glucagon derivative in Embodiment 5 of the present invention.
Fig. 7 shows a schematic representation of the effects of the glucagon derivative on the blood glucose changes of ICR mice in the IPGTT test in Embodiment 6 of the present invention.
Fig. 8 shows a schematic representation of the AUC of blood glucose fluctuation in Embodiment 6 of the present invention.
Fig. 9 shows a schematic representation of the effects of the glucagon derivative on the random blood glucose fluctuation in db/db mice in Embodiment 7 of the present invention.
Fig. 10 shows a schematic representation the effects of the glucagon derivative on the body weight changes of DIO mice in Embodiment 8 of the present invention.

### DETAILED DESCRIPTION OF THE PREFERRED EMBODIMENTS

In order to make the objects, technical solutions and beneficial technical effects of the present invention clearer, the present invention will be further described in detail below in conjunction with the embodiments. Other advantages and effects of the present invention can be easily understood by those who are familiar with the technologies from the contents disclosed in this specification.

After a mass of practice and research, the inventors of the present invention provide a new long-acting glucagon derivative, which has potentially good *in vivo* clinical activity. In animal experiments, the long-acting glucagon derivative effectively regulates the blood glucose level of the subject, reduce the body weights of the subject, so that it can be used for preparing medicines. The present invention is completed on this as basis.

The first aspect of the present invention provides a long-acting glucagon derivative whose amino acid sequence includes a sequence of SEQ ID NO. 9 shown as below:
HX₂QGTFTSDX₁₀SKX₁₃X₁₄DSQAAQDFVQWLLAGGPSSGAPPPS-NH₂ (SEQ ID No. 9);
wherein, X₂ represents Aib (2-amino isobutyric acid group);
X₁₀ represents Y, or K conjugated to a first long-acting carrier;
X₁₃ represents Y, or Aib;
X₁₄ represents L, or K conjugated to a second long-acting carrier;
the chemical structural formula of the 2-amino isobutyric acid group can be shown as below:

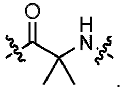

The long-acting glucagon derivative of the present invention is typically prepared from glucagon analogues (GCG analogues) with suitable modifications (e.g., conjugated to long-acting carriers). These GCG analogues may include natural GCG and GCG mutants obtained by amino acid mutation, addition or deletion based on the natural GCG sequence. In a specific embodiment of the present invention, the amino acid sequences of the GCG analogues may include a sequence as shown in one of SEQ ID NOs. 3 to 5. The C-terminal amino acids of the GCG analogues may be modified, for example, may be amidated. Amidation generally means that the -COOH group at C-terminus is transformed into a -CONH₂ group.

**Table 1**

| Compound | Sequence |
|---|---|
| Glucagon | HSQGTFTSDYSKYLDSRRAQDFVQWLMNT (SEQ ID No. 1) |
| GLP-1 | HAEGTFTSDVSSYLEGQAAKEFIAWLVKGRG (SEQ ID No. 2) |
| P13 | H X2QGT FTSDK SKYLD SQAAQDFVQWLLAGGPSSGAPPPS-NH2 X2=Aib (SEQ ID No. 3) |
| P16 | H X2QGT FTSDK SKX13LD SQAAQDFVQWLLAGGPSSGAPPPS-NH2 X2=Aib, X13=Aib (SEQ ID No. 4) |
| P29 | H X2QGT FTSDY SKX13KD SQAAQDFVQWLLAGGPSSGAPPPS-NH2 X2=Aib, X13=Aib (SEQ ID No. 5) |
| GIP | YAEGT FISDY SIAMD KIHQQ DFVNW LLAQK GKKND WKHNI TQ (SEQ ID No. 10) |

In the long-acting glucagon derivative of the present invention, the first long-acting carrier and/or the second long-acting carrier may be a fatty acid chain. The fatty acid chain generally refers to a class of chemical groups consisting of three elements: carbon, hydrogen and oxygen. The fatty acid chain may have a carbon chain with certain length. For example, the length of the carbon chain may be 5-30, 5-6, 6-8, 8-10, 10-15, 15-20, 20-25, or 25-30, preferably 8-30. In a specific embodiment of the present invention, the chemical structural formula of the fatty acid chain may be shown as one of:

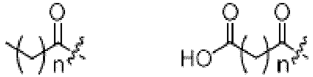

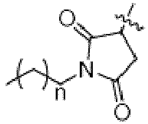

In another specific embodiment of the present invention, each n is independently 3 to 28.

In another specific embodiment of the present invention, each n is independently 6 to 28.

In another specific embodiment of the present invention, each n is independently 17 to 19, e.g., 17, 18, 19.

In the long-acting glucagon derivative of the present invention, the first long-acting carrier and the second long-acting carrier may exist simultaneously or separately. The long-acting carrier is typically conjugated to the amino acid residue (e.g., K) via a linker, thus it can be represented as: -Linker-A, wherein Linker is the linker, and A is the long-acting carrier. In a specific embodiment of the present invention, the linker may be one or more selected from the group consisting of -Abu- (-L-2-aminobutyryl-); -GABA-(-γ-aminobutyryl-); -EACA- (-ε-aminocaproyl-); -β-Ala- (-β-alanyl-); -γGlu-(-γ-glutamyl); -D-γGlu- (-D-γ-glutamyl-) or dipeptide thereof such as -β-Ala-β-Ala-, -γGlu-γGlu- and stereoisomeric forms thereof (S and R enantiomers); -5-Aminopentanoyl-; -8-Aminooctanoyl-); -9-Aminononanoyl-; -10-Aminodecanoyl-; -OEG- (2-(2-(-2-Aminoethoxy)ethoxy)acetyl-); -2xOEG-; -γGlu-OEG-; -γGlu-2xOEG-; -D-γGlu-2xOEG-; -2xOEG-γGlu-; -γGlu-3xOEG-; -γGlu-8xPEG- (-3-((γ-glutamine)-8x polyethylene glycol)-propionyl-); -γGlu-3xOEG-γ-Glu-8xPEG-.

In another specific embodiment of the present invention, the chemical structural formula of the linker may be shown as one of:

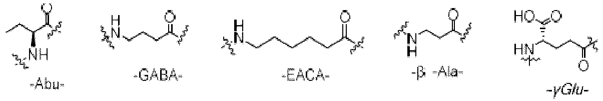

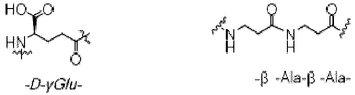

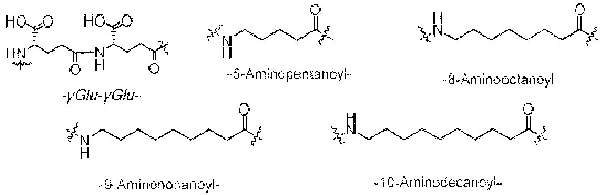

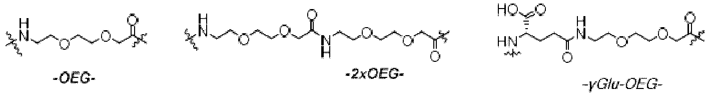

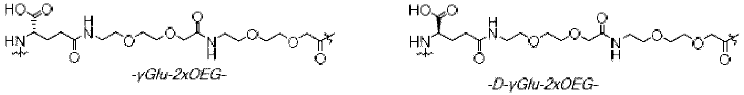

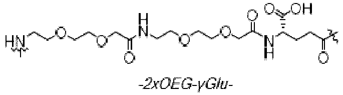

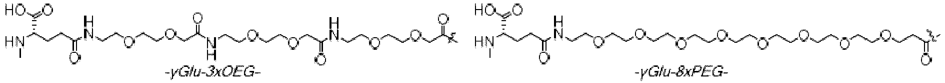

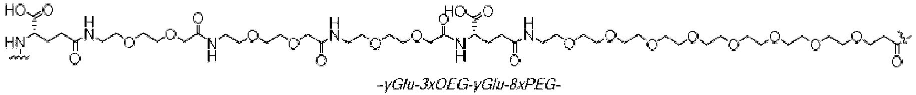

In a specific embodiment of the present invention, X₁₃ represents Y, X₁₄ represents L, X₁₀ represents K conjugated to a fatty acid chain, and the linker is -2xOEG-γGlu-.

In a specific embodiment of the present invention, X₁₃ represents Aib, X₁₄ represents L, X₁₀ represents K conjugated to a fatty acid chain, and the linker is -2xOEG-γGlu-.

In a specific embodiment of the present invention, X₁₃ represents Aib, X₁₀ represents Y, X₁₄ represents K conjugated to a fatty acid chain, and the linker is -2xOEG-γGlu-.

In a specific embodiment of the present invention, the amino acid sequence of the long-acting glucagon derivative includes a sequence as shown in one of SEQ ID Nos. 6 to 8.

HX2QGT FTSDK (2xOEG-γE-COC18H36COOH) SKYLD SQAAQ
DFVQW LLAGG PSSGA PPPS-NH2 X2=Aib (SEQ ID NO: 6)

HX2QGT FTSDK (2xOEG-γE-COC18H36COOH) SKX13LD SQAAQ
DFVQW LLAGG PSSGA PPPS-NH2 X2=Aib X13=Aib (SEQ ID NO: 7)

HX2QGT FTSDY SKX13K (2xOEG-γE-COC18H36COOH) D SQAAQ
DFVQW LLAGG PSSGA PPPS-NH2 X2=Aib X13=Aib (SEQ ID NO: 8)

The second aspect of the present invention provides a method for preparing the long-acting glucagon derivative provided in the first aspect of the present invention, which may include: preparing the long-acting glucagon derivative through solid-phase synthesis. For example, the glucagon derivative can also be prepared in one step through solid-phase chemical synthesis, that is, firstly preparing an amino acid conjugated to a fatty acid chain (e.g., conjugating a fatty acid chain to lysine K), then directly linking the amino acid into a polypeptide chain during the solid-phase chemical synthesis.

The method for preparing the above long-acting glucagon derivative may also include: culturing suitable host cells under suitable conditions to express the above glucagon analogues, separating and purifying to obtain the glucagon analogues, then conjugating fatty acid chains to the glucagon analogues by means of chemical crosslinking, so as to provide the above long-acting glucagon derivative. For example, host cells typically contain constructs including polynucleotides encoding the above glucagon analogues, or a genome integrating exogenous polynucleotides encoding the above glucagon analogues.

The third aspect of the present invention provides a use of the long-acting glucagon derivative provided in the first aspect in preparing a medicament. The long-acting glucagon derivative of the present invention does not have significant advantages in *in vitro* cell-based bioactivity assays (e.g., not showing very high GLP-1R, GIPR or GCGR activity, or the like), but as shown in *in vivo* experiments, they are effective in lowering blood glucose levels in subjects (e.g., disease models (e.g., db/db mice) or normal models), and reducing the body weight of the subjects, so it can be used to prepare the medicament.

In the use of the present invention, the above medicament can be used for treatment of metabolic diseases. The above metabolic diseases are generally diseases associated with insulin abnormal secretion, alternatively for example, the metabolic diseases may be selected from diabetes mellitus, obesity, dyslipidemia, nonalcoholic fatty liver disease (NAFLD), nonalcoholic steatohepatitis (NASH), other diabetes mellitus-related metabolic syndromes, high triglycerides, low HDL cholesterol and high LDL cholesterol, insulin resistance, obesity or glucose intolerance.

The fourth aspect of the present invention provides a pharmaceutical composition, including a therapeutically effective amount of the above long-acting glucagon derivative. The pharmaceutical composition above may also include pharmaceutically acceptable carriers. These carriers may include a variety of excipients and diluents, which are generally not part of essential active ingredients and are not unduly toxic after administration. Suitable carriers should be well known to those skilled in the art. For example, a full discussion of pharmaceutically acceptable carriers can be found in Remington's Pharmaceutical Sciences (Mack Pub. Co., N. J., 1991).

In the present invention, in the medicament or composition above, the above long-acting glucagon derivative can be used as a single effective ingredient, and can also be used in combination with other active components.

The fifth aspect of the present invention provides a therapeutic method including: administering to a subject with a therapeutically effective amount of the long-acting glucagon derivative provided in the first aspect of the present invention or the composition provided in the fourth aspect of the present invention. The therapeutic method of the present invention can be used to treat metabolic diseases or the like.

In the present invention, "subject" generally includes humans, non-human primates, and other mammals, such as dog, cat, horse, sheep, pig, cattle, etc., which can benefit from treatment with the above medicaments, compositions, preparations, kits or combined preparations.

In the present invention, "therapeutically effective amount" generally refers to an amount that can achieve the effect of treating or alleviating the above listed diseases after an appropriate period of administration.

Though the long-acting glucagon derivative of the present invention has low GLP-1R, GIPR agonist activity and weak GCGR agonist activity, superior and more durable glucose tolerance and weight reduction profiles in mice are shown in animal experiments, compared with a similar fatty acid-modified GLP-1R single agonist Semaglutide (see J Med Chem. 2015, 58 (18): 7370-80), which indicating a good industrialization prospect.

The present invention will be further illustrated by the following examples, but the scope of the present invention is not thereby limited.

Unless otherwise stated, the experimental methods, detection methods, and preparative methods disclosed in the present invention all adopt conventional molecular biology, biochemistry, chromatin structure and analysis, analytical chemistry, cell culture, recombinant DNA technologies in the art and conventional techniques in related fields. These techniques are well described in the existing literature and specifically can be found in Sambrook et, al., MOLECULAR CLONING: A LABORATORY MANUAL, Second edition, Cold Spring Harbor Laboratory Press, 1989 and Third edition, 2001; Ausubel et, al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, Chromatin (P. M. Wassarman and A. P. Wolffe, eds.), Academic Press, San Diego, 1999; and METHODS IN MOLECULAR BIOLOGY, Vol. 119, Chromatin Protocols (P. B. Becker, ed.) Humana Press, Totowa, 1999, etc.

The meanings of abbreviations involved in the examples are specifically as follows:
RT: room temperature
DMF: N,N-dimethyl formamide
Fmoc: 9H-fluoren-9-ylmethoxycarbonyl
Trt: trityl
Boc: tert-butoxycarbonyl
HOBt: 1-hydroxybenzotriazole
tBu: tert-butyl
DCM: dichloromethane
DBLK: 20% N,N-dimethylformamide piperidine
DIC: N,N'-diisopropylcarbodiimide
MeOH: methanol
TFA: trifluoroacetic acid
Aib: 2-aminoisobutyric acid
TFEA: 2,2,2-trifluoroethanol
Pd(PPh₃)₄: tetrakis(triphenylphosphine)palladium
Alloc: allyloxycarbonyl
-OEG-: -2-(2-(2-aminoethoxy)ethoxy)acetyl-
-γE-: -γ-glutamyl-
DMAP: dimethylaminopyridine
DIEA: N,N-diisopropylethylamine
MTBE: methyl tert-butyl ether

For various commercially available amino acids and amino acid fragments, as well as various commercially available resins involved in the embodiments, their manufacturers and product models are as follows:

Fmoc-protected amino acid raw materials, 2-CTC resin and Wang Resin are all conventional commercial reagents (Manufacturer of protected amino acids: Chengdu ZY Biochemical Technology Co., Ltd.; Manufacturer of resins: Tianjin Nankai HECHENG S&T Co., Ltd.);

Organic solvents and other raw materials are all commercially available (Manufacturer: Sinopharm Chemical Reagent limited corporation; chemically pure).

Additionally, conditions for HPLC and mass spectrometry as well as models and manufacturers of equipment used are illustrated as follows:

Instrument: HPLC UltiMate 3000 (Thermo Fisher); the detection conditions are shown in Table 2 below.

**Table 2**

| | | | | | | | |
|---|---|---|---|---|---|---|---|
| Chromato graphic column | Jupiter^{®} 4 um Proteo 90 Å 4.6*250 mm | | | | | | |
| Mobile phase | A: TFA: purified water = 1 :1000 | | | | | | |
| | B: TFA: acetonitrile = 1:1000 | | | | | | |
| Detection parameter s | Flow rate: 1.0 ml/min, Wavelength: 220 nm, Column temperature: 45°C ..... | | | | | | |
| Elution gradient | T (min) | 0 | 40 | 41 | 46 | 47 | 55 |
| | A (%) | 75 | 35 | 10 | 10 | 75 | 75 |
| | B (%) | 25 | 65 | 90 | 90 | 25 | 25 |

Preparative liquid phase: Beijing Tong Heng Innovation Technology Co., Ltd., LC3000.

Mass spectrometry: Instrument model is 5800 MALDI-TOF-TOF (AB SCIEX), the analysis software is TOF/TOF Explorer, Data Explorer, MS employs Reflector Positive parameters: CID(OFF), mass rang (700-6500 Da) Focus Mass (1200Da) Fixed laser intensity (5600) Digitizer: Bin Size (0.5 ns).

### Embodiment 1

### Synthesis of branched-chain-protected amino acid W2

The structure of Alloc-Lys ((Octadecanedioic Acid mono-tert-butylester)-Glu-OtBu)-OEG-OEG)-OH is as below:

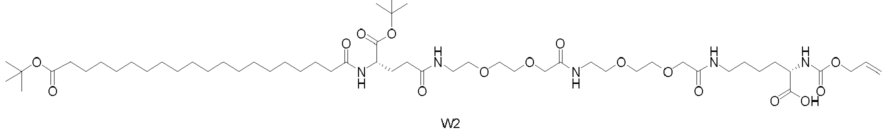

### Synthesis of W2:

20 g of 2-CTC resin (Tianjin Nankai HECHENG S&T Co., Ltd., Item no. G55W0509) with substitution of 1.0 mmol/g was weighed, added into a solid-phase reaction column, and washed once with DMF. After swelling resin with DMF for 30 minutes, 8.53 g Alloc-Lys(Fmoc)-OH (20 mmol) was dissolved in DMF, activated by adding 7.5 ml DIEA (45 mmol) under an ice-water bath, and then loaded into the above reaction column filled with resin. After reaction for 2 hours, 30 ml anhydrous methanol was added to end-cap for 1 hour, and then washed with DMF for 3 times. After removing Fmoc protection with a mixed solution of DMF and piperidine at a volume ratio of 4:1, and washing with DMF for 6 times, 15.42 g [2-[2-(Fmoc-amino) ethoxy] ethoxy] acetic acid and 5.41 g HOBt were dissolved in DMF, activated by adding with 6.2 ml DIC under an ice-water bath, and then loaded into the above reaction column filled with resin and reacted at room temperature for 2 hours. By repeating the above steps of removing Fmoc protection and adding corresponding materials for coupling, [2-[2-(Fmoc-amino)ethoxy]ethoxy]acetic acid, Fmoc-Glu-OtBu, and octadecanedioic acid mono-tert-butyl ester were coupled in turns according to the order of branched-chain fragments. After the completion of coupling, the resin was washed with DMF for 3 times, washed with MeOH for 5 times, and drained. The resin was added into 400 ml of TFEA/DCM = 1:4 to react at room temperature for 4 h. After the resin was filtered, the filtrate was spun to remove DCM, then added into 500 ml MTBE for settlement, and finally, centrifuged and dried to get a target compound of 20.12 g.

### Embodiment 2

### Preparation of a glucagon derivative-P13F:

The structural formula of the glucagon derivative P13F is as below: 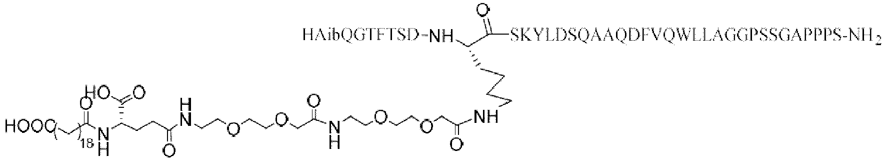 that is: HAibQGT FTSD K (2xOEG-γE-COC₁₈H₃₆COOH) SKYL D SQAAQ DFVQW LLAGG PSSGA PPPS-NH₂ (SEQ ID NO: 6)

### Synthesis of peptide resin:

2.85 g of Rink amide MBHA resin (Tianjin Nankai HECHENG S&T Co., Ltd., GRMS1217) with substitution of 0.35 mmol/g was weighed, added into a solid-phase reaction column, swelled with 20 mL DCM in the reaction column for 30 minutes, and then washed with DMF for 3 times, with 20 mL for each time. After washing, 10 mL DBLK solution (20% piperidine/DMF(V/V)) was added into the reaction column to react for 5 minutes, then suction filtrated, and washed once with 20 mL DMF. 10 mL DBLK solution (20% piperidine/DMF(V/V)) was additionally added to react for 10 minutes, and detected by Kaiser as positive. The reaction solution was suction filtrated, and washed with DMF for 3 times, with 20 mL for each time. Fmoc-Ser (tBu)-OH (1.91 g, 5.0 eq) and HOBt (0.81 g, 6.0 eq) were additionally added into 10 mL DMF to dissolve, and activated by adding DIC (0.69 g, 5.5 eq) for 5 min at 5-8°C, they were then added into the reaction column to react for 1 hour, and then detected by Kaiser as negative. Upon completion, the reaction was washed with DMF for 3 times, with 20 mL for each time. By repeating the above deprotection and coupling operations, other amino acids were coupled in turns according to the amino acid sequence of the polypeptide, wherein X₁₀ was coupled with W2, and the Alloc group was removed by Pd(PPh₃)₄. After the completion of the coupling of the last amino acid, deprotection was conducted following the above deprotection method. After the completion of deprotection, it was successively washed with DMF twice, washed with MeOH twice, washed with DCM twice and washed with MeOH twice, with 20 mL for each washing solvent. The materials were collected, dried at normal temperature and at reduced pressure to get the target peptide resin.

### Cleavage of crude peptide:

4.93 g of the above peptide resin was weighed, slowly added into 60 mL cleavage cocktail (trifluoroacetic acid: thioanisole: anisole: ethanedithiol=90:5:3:2) at 20-30°C, and reacted for 2 hours after the completion of addition. Upon the completion of reaction, resin was removed by filtration. The filtrate was poured into pre-cooled methyl tert-butyl ether (600mL) under vigorous stirring. The resulting mixed solution was placed in a refrigerator to settle for 2 hours. After removing the supernatant, the solution was washed with pre-cooled methyl tert-butyl ether for 5 times by centrifugation, with 400 mL for each time. After completion, the materials were collected, dried at normal temperature and at reduced pressure to get 2.37 g crude peptides.

### Purification of crude peptides:

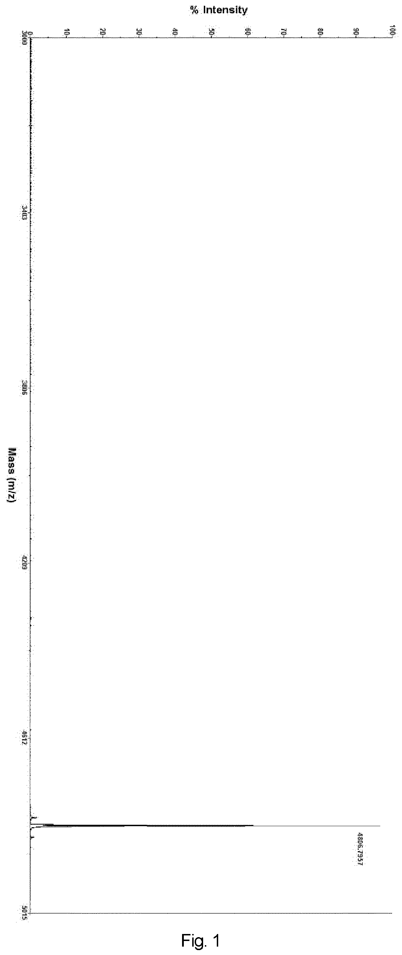

The crude peptides were refined by multi-step purification using preparative liquid phase (Beijing Tong Heng Innovation Technology Co., Ltd., LC3000): Step 1: stationary phase: C18 (Daisogel: sp-120-40/60-C18-RPS), mobile phase: 0.1% TFA, acetonitrile; Step 2: stationary phase: C8 (Daisogel: sp-120-10-C8-P), mobile phase: 0.5% phosphoric acid, acetonitrile; Step 3: stationary phase: C8 (Daisogel: sp-120-10-C8-P), mobile phase: 50 mM ammonium acetate, 0.3% acetic acid, acetonitrile, and finally lyophilized (using a lyophilizer commercially available from Beijing boyikang Lab Instrument Co., Ltd., FD-2A) to get fine peptide (97.1%). Mass spectrometry (Ms) detection results m/z 4806.7957 (M+H)⁺ were shown in Fig. 1.

### Embodiment 3

### Preparation of a glucagon derivative-P16F:

The structural formula of the glucagon derivative P16F is as below: 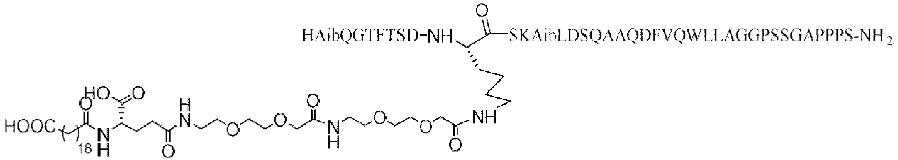 that is: HAibQGT FTSD K (2xOEG-γE-COC₁₈H₃₆COOH) SKAibL D SQAAQ DFVQW LLAGG PSSGA PPPS-NH₂ (SEQ ID NO. 7)

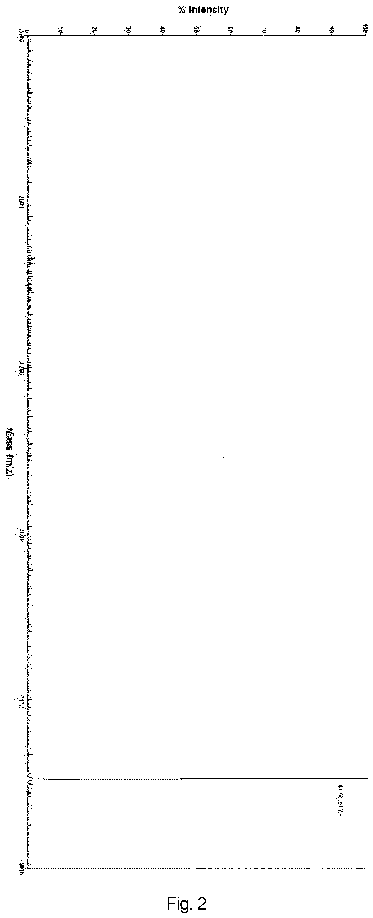

The synthesis of polypeptides was the same as that of P13F, wherein X₁₀ was coupled with W2, and the Alloc group was removed by Pd(PPh₃)₄. The resulting crude peptide was purified by RP-HPLC to get the fine peptide (95.3%). MS detection results: m/z 4728.6129 (M+H)⁺ were shown in Fig. 2.

### Embodiment 4

### Preparation of a glucagon derivative-P29F:

The structural formula of the glucagon derivative P29F is as below: 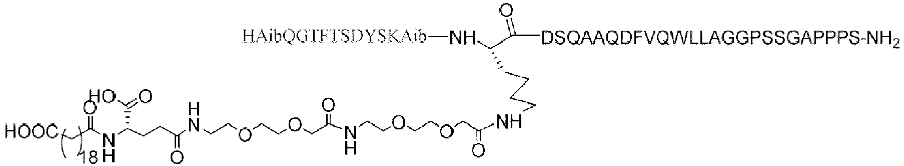 that is: HAibQGT FTSDY SKAib K (2xOEG-γE-COC₁₈H₃₆COOH) D SQAAQ DFVQW LLAGG PSSGA PPPS-NH₂ (SEQ ID NO. 8)

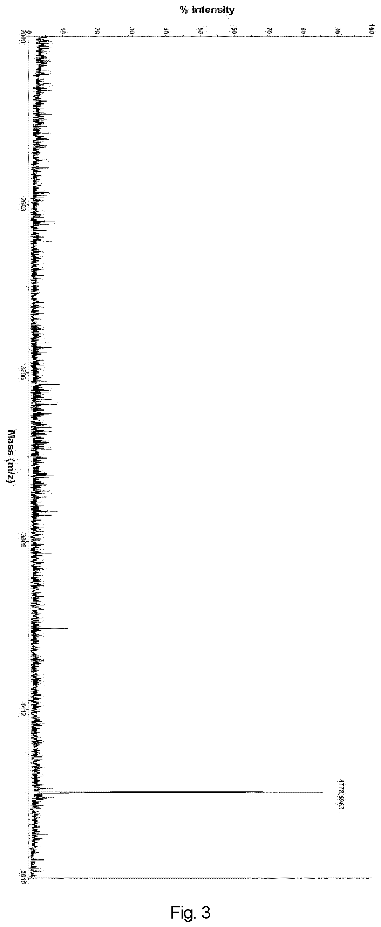

The synthesis of polypeptides was the same as that of P13F, wherein X₁₄ was coupled with W2, and the Alloc group was removed by Pd(PPh₃)₄. The resulting crude peptide was purified by RP-HPLC to get the fine peptide (96.0%). MS detection results: m/z 4778.5963 (M+H)⁺ were shown in Fig. 3.

### Embodiment 5

### In vitro cell-based bioactivity assay:

### I. GLP-1R agonist activity assay:

GLP-1R agonist activity was detected using luciferase reporter gene assay (Jonathan W Day et, al.: Nat Chem Biol. 2009 Oct; 5 (10): 749-57). Humanized GLP-1R gene (NM_002062.5) was cloned into mammalian cell expression plasmid pCDNA3.1 to construct recombinant expression plasmid pCDNA3.1-GLP-1R, and at the same time, luciferase full-length gene (XM_031473197.1) was cloned into pCRE plasmid to obtain pCRE-Luc recombinant plasmid. The pcDNA3.1-GLP-1R and pCRE-Luc plasmids were transfected into CHO-K1 cells at a molar ratio of 1:10, and the stably transfected strains were screened.

Cells were cultured in 9-cm cell culture dishes with DMEM/F12 medium containing 10% FBS and 300 µg/ml G418. When the confluence reached about 90%, the culture supernatant was discarded. After digestion with 2 ml of trypsin for 3 min, 2 ml of DMEM/F12 medium containing 10% FBS and 300 µg/ml G418 was added for neutralization, and then transferred into 15 ml centrifuge tubes. After centrifugation at 1000 rpm for 5 min, the supernatant was discarded. 2 ml of DMEM/F12 medium containing 10% FBS and 300 µg/ml G418 was added for cell resuspension, and the cells were counted. Cells were diluted with DMEM/F12 medium containing 10% FBS to 1×10⁵ cells/ml, and plated in 96-well plates at 100 µl/well, that is 1×10⁴ cells/well. After adherence, the medium was replaced with DMEM/F12 medium containing 0.2% FBS for culture. After discarding the supernatant from the cells plated in the 96-well plates, the purified proteins (P13F, P16F, P29F) or control polypeptide proteins: GLP-1 (GLUC-014, Chinese Peptide Company), GIP (GIPS-001, Chinese Peptide Company) or GCG (GLUC-004, Chinese Peptide Company) were diluted with DMEM/F12 medium containing 1% BSA to a series of defined concentration and pipetted into the plates at 100 µl/well. After stimulation for 6 hours, luminescence signals were detected according to the protocol of Bright-Glo^{™} Luciferase Assay System (Promega, E2620). The assay was repeated 3 times for each sample.

### II. GIPR agonist activity assay:

GIPR agonist activity was also detected using luciferase reporter gene assay. Humanized GIPR gene (NM_000164) was cloned into mammalian cell expression plasmid pcDNA3.1 to construct recombinant expression plasmid pCDNA3.1-GIPR, and transfected into CHO-K1 cells. The screening of the stably transfected strains was the same as above. The assay was repeated 3 times for each sample.

### III. GCGR agonist activity assay:

GCGR agonist activity was also detected using luciferase reporter gene assay. Humanized GCGR gene (NM_000160) was cloned into mammalian cell expression plasmid pcDNA3.1 to construct recombinant expression plasmid pCDNA3.1-GCGR, and transfected into CHO-K1. The screening of the stably transfected strains was the same as above. The assay was repeated 3 times for each sample.

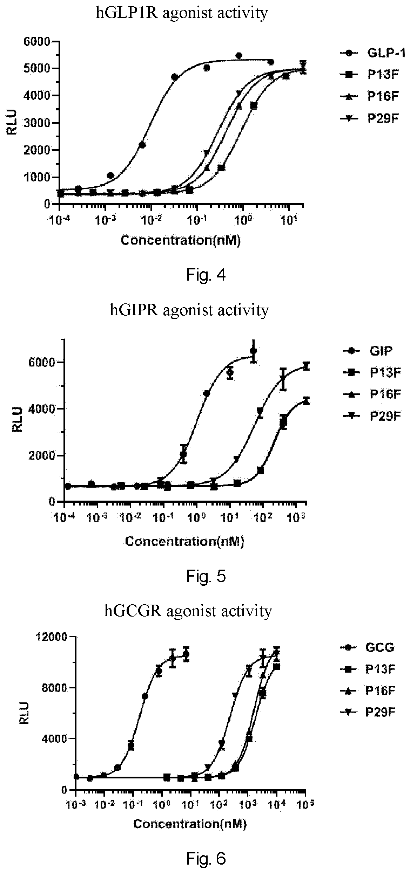

The cell-based assay results are shown in Fig. 4 to Fig. 6, and the corresponding EC50 values are shown in Table 3.

The data shows that: P13F, P16F and P29F all have low GLP-1R (about 1%-3% that of natural GLP-1 peptide) and GIPR agonist activities (about 0.5%-2% that of natural GIP peptide), and minimal GCGR agonist activity. P29 shows the highest, about 2% of natural GIP, GIPR agonist activity.

**Table 3**

| Code of active protein | GLP-1R agonist activity (EC50, nM) | GIPR agonist activity (EC50, nM) | GCGR agonist activity (EC50, nM) |
|---|---|---|---|
| GLP-1 | 0.009 | / | / |
| GCG | / | / | 0.17 |
| GIP | / | 1.03 | / |
| P13F | 0.90 | 220.20 | >1000 |
| P16F | 0.43 | 211.70 | >1000 |
| P29F | 0.28 | 55.61 | 243.90 |

### Embodiment 6

### In vivo IPGTT experiment in normal ICR mice:

After the quarantine acclimation period, the animals were fasted for about 10 hours, and blood was collected from the tip of the tail to measure the fasting blood glucose. Then 20% glucose (2 g/kg) was injected intraperitoneally, and blood glucose was measured 0.5 h after injection. Animals were screened according to the glycemic increase levels. The animals whose increased glycemic level deviated from the average after glucose administration were excluded. 40 animals were screened for the following experiment.

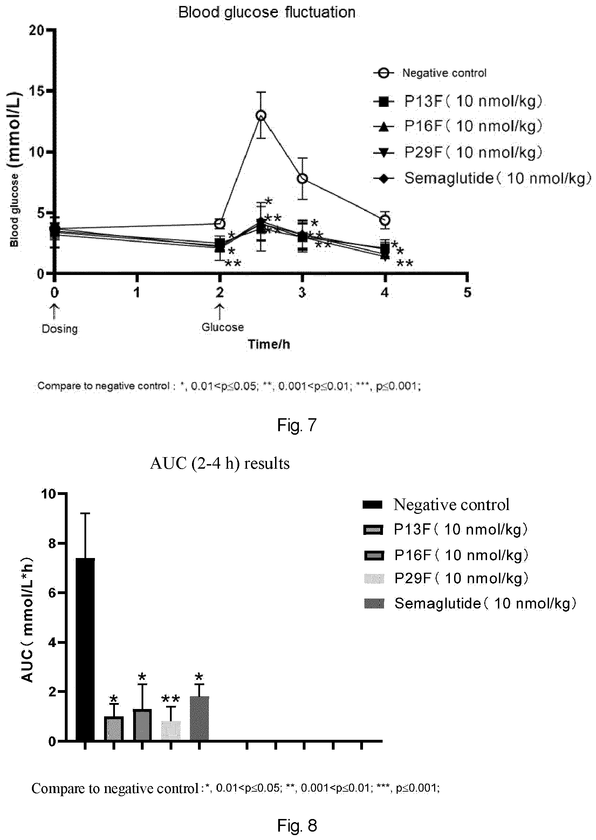

ICR mice were divided into 5 groups based on their body weight, with 8 mice in each group, and subjected to formal experiment after adaptive feeding in separate cages for 2 days. After fasting for 10 hours (T=0 h), P13F, P16F, P29F and the positive control Semaglutide were subcutaneously administered, all at a dose of 10 nmol/kg. Saline PBS was injected (5 ul/g body weight) as the negative control. 2 hours (T=2 h) later, 20% glucose was injected (2 g/kg body weight) intraperitoneally. At different time points, i.e., before injection of glucose and 30, 60 and 120 minutes (T=2.5 h, 3 h, 4 h) after injection of glucose, the blood glucose were measured, with the results shown in Figs. 7 and 8. It can be inferred from the figures that, P13F, P16F and P29F are superior in lowering blood glucose and controlling blood glucose, compared with Semaglutide (Novo Nordisk A/S).

### Embodiment 7

### Random blood glucose in db/db mice after administration:

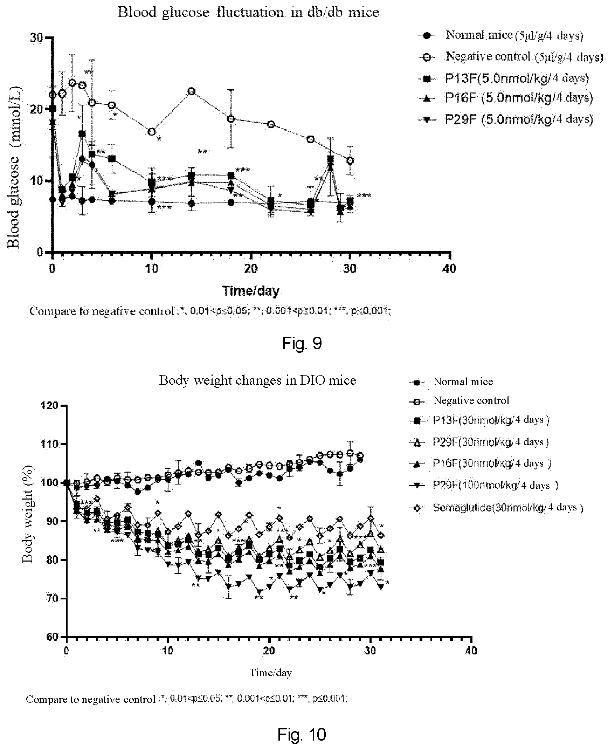

Leptin receptor deficiency type II diabetes mellitus (db/db) mice were screened and grouped evenly according to three indicators: body weight, non-fasting blood glucose, and pre-drug OGTT response, with 8 mice in each group, individuals that were too large or too small were excluded, and their non-fasting blood glucose should be greater than 15 mM. P13F, P16F and P29F were dissolved in 50 mM phosphate buffer (pH 7.4) containing 5% sorbitol and 0.02% v/v Tween-80. The negative control PBS or the glucagon derivatives were administrated subcutaneously. The glucagon derivatives were administrated in stages at different doses of 5.0 nmol/kg/4 days (30 days). A control group of normal mice was included as well, which were injected with the negative control PBS (5 µl/g body weight) subcutaneously. During days 0-4, random blood glucose values were recorded in all animals every day, and then on day 6, and subsequently recorded once every 4 days, which scheduled on days 6, 10, 14, 18, 22, 26; and days 28, 29, 30. The profile of random blood glucose fluctuation was shown in Fig. 9.

The results showed that the blood glucose levels of the mice injected with P13F, P16F and P29F decreased to normal at day 22, and maintained until day 30.

### Embodiment 8

### Weight loss in diet-induced obesity (DIO) mice:

Preparation of DIO mouse models: Male C57BL/6J mice of about 7-week-old were fed a high-fat diet (60% kcal from fat) for about 16 weeks (totally 23 weeks), until the body weight reached to about 45 g. DIO mice were randomly grouped, with 6 mice in each group and no difference in basal body weight. Body weights were recorded every day. P13F, P16F, P29F, the positive control Semaglutide, or the negative control PBS were injected subcutaneously. P29F were administrated at 30 nmol/kg/4 days and 100 nmol/kg/4 days; while P13F, P16F and the positive control Semaglutide were administrated at 30 nmol/kg/4 days. Body weights were recorded on the first day of administration, until the end of the experiment Day 30. The food intake and body weights were recorded consistently every day. The results are shown in Fig. 10.

According to the results, P13F, P16F and P29F administrated at a dose of 30 nmol/kg/4 days show superior effects on weight loss, compared to Semaglutide. All the dosing groups reached the maximum weight loss at around day 20, and maintained until around day 30. Additionally, in terms of P29F, weight loss was dose dependent; when it was administrated at a dose of 100 nmol/kg/4 days, the weight loss proportion at day 30 approached 30%. It is demonstrated that, although the glucagon derivatives of the present invention do not have very high GLP-1R, GIPR or GCGR agonist activity, their *in vivo* efficacy are very obvious. In particular, GLP-1R agonist activity is highly correlated with gastrointestinal side effects, and the property of low activity becomes the potential advantage of the present invention, which is completely different from the existing preclinical or clinical candidates for highly active poly-agonists.

In summary, the present invention effectively overcomes various disadvantages in the prior art and has high industrial utilization value.

The above embodiments are only intended to illustrate the principles and effects of the present invention, but are not to limit the present invention. Anyone skilled in the art can modify or change the above embodiments without departing from the spirit and scope of the present invention. Therefore, all equivalent modifications or changes made by those with ordinary knowledge in the technical field without departing from the spirit and technical idea disclosed in the present invention shall still be encompassed by the claims of the present invention.

## Claims

1. A long-acting glucagon derivative, comprising a sequence as shown in SEQ ID NO. 9:
HX₂QGTFTSDX₁₀SKX₁₃X₁₄DSQAAQDFVQWLLAGGPSSGAPPPS-NH₂ SEQ ID No. 9;
wherein, X₂ represents Aib;
X₁₀ represents Y, or K conjugated to a first long-acting carrier;
X₁₃ represents Y, or Aib;
X₁₄ represents L, or K conjugated to a second long-acting carrier.

2. The long-acting glucagon derivative according to claim 1, wherein the first long-acting carrier and/or the second long-acting carrier is a fatty acid chain.

3. The long-acting glucagon derivative according to claim 2, wherein the carbon-chain length of the fatty acid chain is 5-30, preferably 8-30;
and/or, the chemical structural formula of the fatty acid chain is shown as one of: 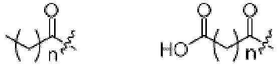 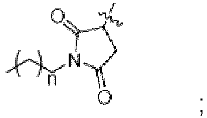
wherein, each n is independently 3 to 28, preferably 6 to 28, more preferably 17, 18, or 19.

4. The long-acting glucagon derivative according to claim 2, wherein the fatty acid chain is conjugated to K via a linker, the linker is selected one or more from the group consisting of -Abu-, -GABA-, -EACA-, -β-Ala-, -γGlu-, -D-γGlu- or dipeptide thereof, -β-Ala-β-Ala-, -γGlu-γGlu- and stereoisomeric forms thereof, -5-Aminopentanoyl-, -8-Aminooctanoyl-, -9-Aminononanoyl-, -10-Aminodecanoyl-, -OEG-, -2xOEG-, -γGlu-OEG-, -γGlu-2xOEG-, -D-γGlu-2xOEG-, -2xOEG-γGlu-, -γGlu-3xOEG-, -γGlu-8xPEG-, -γGlu-3xOEG-γ-Glu-8xPEG-.

5. The long-acting glucagon derivative according to claim 2, wherein X₁₃ represents Y, X₁₄ represents L, X₁₀ represents K conjugated to a fatty acid chain, and the linker is -2xOEG-γGlu-;
and/or, X₁₃ represents Aib, X₁₄ represents L, X₁₀ represents K conjugated to a fatty acid chain, and the linker is -2xOEG-γGlu-;
and/or, X₁₃ represents Aib, X₁₀ represents Y, X₁₄ represents K conjugated to a fatty acid chain, and the linker is -2xOEG-γGlu-.

6. The long-acting glucagon derivative according to claim 2, wherein the amino acid sequence of the long-acting glucagon derivative comprises a sequence as shown in one of SEQ ID Nos. 6 to 8.

7. A method for preparing the long-acting glucagon derivative according to any one of claims 1 to 6, comprising: preparing the long-acting glucagon derivative through solid-phase synthesis.

8. A use of the long-acting glucagon derivative according to any one of claims 1 to 6 in preparing a medicament, wherein the medicament is preferably a medicament used for treating a metabolic disease.

9. The use according to claim 8, wherein the metabolic disease is selected from diabetes mellitus, obesity, dyslipidemia, nonalcoholic fatty liver disease, nonalcoholic steatohepatitis, other diabetes mellitus-related metabolic syndromes, high triglycerides, low HDL cholesterol and high LDL cholesterol, insulin resistance, obesity or glucose intolerance.

10. A pharmaceutical composition, comprising a therapeutically effective amount of the long-acting glucagon derivative according to any one of claims 1 to 6.
